Europäisches Patentamt

(19) European Patent Office

Office européen des brevets

(11) Publication number: **0 109 748**
**B1**

(12) # EUROPEAN PATENT SPECIFICATION

(45) Date of publication of patent specification: **13.04.88**

(21) Application number: **83306221.9**

(22) Date of filing: **13.10.83**

(51) Int. Cl.⁴: **C 12 N 15/00, C 12 P 21/02, A 61 K 37/02**

(54) **Pharmaceutical and veterinary preparations of cystein-125-depleted muteins of Interleukin-2 and their production.**

(30) Priority: **19.10.82 US 435154**
**15.04.83 US 486162**

(43) Date of publication of application:
**30.05.84 Bulletin 84/22**

(45) Publication of the grant of the patent:
**13.04.88 Bulletin 88/15**

(84) Designated Contracting States:
**AT CH DE FR IT LI LU NL SE**

(56) References cited:
**EP-A-0 136 489**
**US-A-4 399 216**
**J. Mol. Appl. Genet. (1981), pages 165-175**
**PROC. NATL. ACAD. SCI. (USA), 1982, vol. 79, pages 4897-4901**
**DNA (1984), vol. 3, pages 297-308**
**NATURE, vol. 294, no. 5841, December 10, 1981, (New York, USA) H.M. SHEPARD et al.:**

**The file contains technical information submitted after the application was filed and not included in this specification**

(73) Proprietor: **CETUS CORPORATION**
**1400 Fifty-Third Street**
**Emeryville California 94608 (US)**

(72) Inventor: **Mark, David F.**
**217 Standbridge Ct.**
**Danville, CA 94526 (US)**
Inventor: **Lin, Leo S.**
**40880 Los Pinos**
**Fremont, CA 94539 (US)**
Inventor: **Yu Lu, Shi-Da**
**366 Euclid Avenue 109**
**Oakland, Ca 94610 (US)**

(74) Representative: **Bizley, Richard Edward et al BOULT, WADE & TENNANT 27 Furnival Street London EC4A 1PQ (GB)**

(56) References cited:
**"A single amino acid change in IFN-beta1 abolishes its antiviral activity" pages 563-565**

Courier Press, Leamington Spa, England.

## Description

This invention is in the general area of recombinant DNA technology. More specifically it relates to mutationally altered biologically active interleukin-2 proteins that differ from their parent analog by substitution of cysteine residues.

Biologically active proteins that are microbially produced via recombinant DNA (rDNA) technology may contain cysteine residues that are nonessential to their activity but are free to form undesirable intermolecular or intramolecular links.

Human IL-2 is reported to have three cysteine residues located at positions 58, 105, and 125 of the protein. IL-2 is in an aggregated oligomeric form when isolated from bacterial cells and has to be reduced with reducing agents in order to obtain a good yield from bacterial extracts. In addition, the purified reduced IL-2 protein is unstable and readily reoxidized upon storage to an oligomeric inactive form. The presence of three cysteines means that upon reoxidation, the protein may randomly form one of three possible intramolecular disulfide bridges, with only one of those being the correct bridge as found in the native molecule.

The present invention is directed to producing by directed mutagenesis techniques mutationally altered biologically active IL-2 proteins (such proteins are called "muteins", *Glossary of Genetics and Cytogenetics*, 4th Ed, p 381, Springer-Verlag (1976)) that retain the activity of their parent analogs but lack the ability to form intermolecular links or undesirable intramolecular disulfide bonds. In this regard Shepard, H. M. et al, *Nature* (1981) *294*:563—565 describe a mutein of IFN-β in which the cysteine at position 141 of its amino acid sequence (there are three cysteins in native human IFN-β at positions 17, 31, and 141, *Gene* (1980) *10*:11—15 and *Nature* (1980) 285:542—547) is replaced by tyrosine. This mutein was made by bacterial expression of a hybrid gene constructed from a partial IFN-β cDNA clone having a G→A transistion at nucleotide 485 of the IFN-β gene. The mutein lacked the biological activity of native IFN-β leading the authors to conclude that the replaced cysteine was essential to activity.

Directed mutagenesis techniques are well known and have been reviewed by Lather, R. F. and Lecoq. J. P. in *Genetic Engineering* Academic Press (1983) pp 31—50. Oligonucleotide-directed mutagenesis is specifically reviewed by Smith, M. and Gillam, S. in *Genetic Engineering: Principles and Methods*, Plenum Press (1981) *3*:1—32.

The European Patent Application EP—A—136489 (published on 10.4.85; filing date 10.8.84; priority dates 3.8.84 and 10.8.83) discloses a number of possible aminoacid substitutions in interleukin-2, among them also the replacement of the cysteine residue in position 125 with alanine or serine.

The invention provides a biologically active recombinant human interleukin-2 mutein pharmaceutical or veterinary preparation suitable for use in therapeutic or diagnostic applications, which preparation is incapable of forming an improper intramolecular disulfide bridge and is stable upon storage relative to bacterially derived reduced native interleukin-2, wherein the cysteine residue normally present at position 125, numbered in accordance with native interleukin-2, (see Taniguchi et al, Nature (1983) Vol. 302, p. 305 et seq), that is free to form a disulfide link and is non-essential to said biological activity has been substituted by a neutral amino acid.

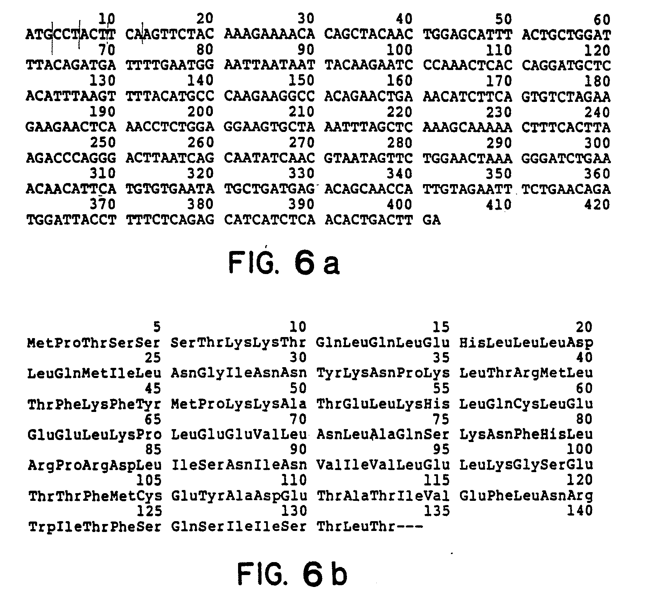

Another aspect of the invention is synthetic genes having DNA sequences that have been specifically designed ("designer genes") to encode muteins as described above which have an amino acid sequence as shown in Figure 6b of the accompanying drawings, optionally in which the serine residue in position 125, numbered in accordance with native interleukin-2, is an alternative neutral amino acid other than cysteine (see later). Other aspects are expression vectors that include such designer genes, host cells or organisms transformed with such vectors, and processes for making recombinant mutein preparations by culturing such transformants or their progeny and recovering the mutein from the culture. Therapeutic formulations whether for human or veterinary use (and optionally in unit dosage form) are another aspect of the invention.

Another aspect of the invention is a method of preventing a protein having one or more cysteine residues that is free to form an undesirable disulfide link from forming such a link characterized in that the protein is mutationally altered by deleting the cysteine residue(s) or replacing it/them with other amino acids.

Still another aspect of the invention is a method for making the above described synthetic structural gene by oligonucleotide-directed mutagenesis comprising the following steps:

(a) hybridizing single-stranded DNA comprising a strand of a structural gene that encodes the parent protein with a mutant oligonucleotide primer that is complementary to a region of the strand that includes the codon for the cysteine to be deleted or replaced or the antisense triplet paired with the codon, as the case may be, except for a mismatch with that codon or antisense triplet, as the case may be, that defines a deletion of the codon or a triplet that encodes said other amino acid:

(b) extending the primer with DNA polymerase to form a mutational heteroduplex; and

(c) replicating the mutational heteroduplex.

The mutant oligonucleotide primers used in this process are another aspect of the invention.

Reference is made to copending European Patent Application No. 85109295.7 (EP—A—192811), a divisional application from this present application. Said divisional application claims the general principle of a recombinant synthetic mutein of a biologically active protein normally having at least one cysteine residue that is free to form a disulfide link and is non-essential to said biological activity in which at least one of said cysteine residues has been deleted or replaced by another amino acid.

Reference is also made to co-pending European Patent Application No. 86110595.5, which is divisional application from this present application which claims muteins of interferon-β in which the cysteine residue at at position 17 is deleted or replaced by another amino acid.

The invention will now be further described and illustrated with reference to the accompanying drawings, in which:—

Figure 1 is a diagram of the cloning vector M13mp8 phage;

Figure 2 is a diagram of the expression plasmid pTrp3;

Figure 3 is a diagram of the plasmid pLW1 which contains the human interleukin-2 (IL-2) gene under the control of the *E. coli* trp promoter;

Figure 4 is a restriction map of phage clone M13—IL2;

Figure 5 is a restriction map of the plasmid pLW46; and

Figures 6a and 6b show, respectively, the nucleotide sequence of the coding strand of the clone pLW46 and the corresponding amino acid sequence of the IL-2 mutein designated IL-2$_{ser125}$.

In human IL-2, since the disulfide structure of the native IL-2 protein is not known, it is possible to use the present invention to create mutations at codons 58, 105 and 125 of the IL-2 gene and identify which cysteine residues are necessary for activity and therefore most likely to be involved in native disulfide bridge formation. In the same vein, the cysteine residue that is not necessary for activity can be modified so as to prevent the formation of incorrect intramolecular disulfide bridges and minimize the chance of intermolecular disulfide bridges by replacement of the free cysteine residue.

By the use of the oligonucleotide-directed mutagenesis procedure with a synthetic oligonucleotide primer that is complementary to the region of the IL-2 gene at the codon for cys 125 but which contains single or multiple base changes in that codon, a designer gene may be produced that results in cys 125 being replaced with any other amino acid of choice.

Conversion of cys 125 to neutral amino acids such as glycine, valine, alanine, leucine, isoleucine, tyrosine, phenylalanine, histidine, tryptophan, serine, threonine and methionine is the preferred approach. Serine and threonine are the most preferred replacements because of their chemical analogy to cysteine.

The size of the oligonucleotide primer is determined by the requirement for stable hybridization of the primer to the region of the gene in which the mutation is to be induced, and by the limitations of the currently available methods for synthesizing oligonucleotides. The factors to be considered in designing oligonucleotides for use in oligonucleotide-directed mutagenesis (e.g. overall size, size of portions flanking the mutation site) are described by Smith, M. and Gillam S., supra. In general the overall length of the oligonucleotide will be such as to optimize stable, unique hybridization at the mutation site with the 5' and 3' extensions from the mutation site being of sufficient size to avoid editing of the mutation by the exonuclease activity of the DNA polymerase. Oligonucleotides used for mutagenesis in accordance with the present invention usually contain from about 12 to about 24 bases, preferably from about 14 to about 20 bases and still more preferably from about 15 to about 18 bases. They will usually contain at least about three bases 3' of the altered or missing codon.

Oligonucleotide-directed mutagenesis may be employed to make a mutant IL-2 gene that encodes a mutein having IL-2 activity but having cys 125 changed to serine 125. The preferred oligonucleotide primer used in making this mutant IL-2 gene when the phage carries the sense strand of the gene is GATGATGCTTCTGAGAAAAGGTAATC. This oligonucleotide has a C→G change at the middle base on the triplet that is paired with codon 125 of the IL-2 gene.

It must be recognized that the genetic code is degenerate and that many of the amino acids may be encoded by more than one codon. The base code for serine, for example, is six-way degenerate such that the codons, TCT, TCG, TCC, TCA, AGT, and ACG all code for serine. Similarly, threonine is encoded by any one of codons ACT, ACA, ACC and ACG. It is intended that when one codon is specified for a particular amino acid, it includes all degenerate codons which encode that amino acid.

The primer is hybridized to single-stranded phage such as M13, fd, or Ø×174 into which a strand of the IL-2 gene has been cloned. It will be appreciated that the phage may carry either the sense strand or antisense strand of the gene. When the phage carries the antisense strand the primer is identical to the region of the sense strand that contains the codon to be mutated except for a mismatch with that codon that defines a deletion of the codon or a triplet that codes for another amino acid. When the phage carries the sense strand the primer is complementary to the region of the sense strand that contains the codon to be mutated except for an appropriate mismatch in the triplet that is paired with the codon to be deleted. Conditions that may be used in the hydridization are described by Smith, M. and Gillam, S., supra. The temperature will usually range between about 0°C and 70°C, more usually about 10°C to 50°C. After the hybridization, the primer is extended on the phage DNA by reaction with DNA polymerase I, T$_4$ DNA polymerase, reverse transcriptase or other suitable DNA polymerase. The resulting dsDNA is converted to closed circular dsDNA by treatment with a DNA ligase such as T$_4$ DNA ligase. DNA molecules containing single-stranded regions may be destroyed by S1 endonuclease treatment.

The resulting mutational heteroduplex is then used to transform a competent host organism or cell. Replication of the heteroduplex by the host provides progeny from both strands. Following replication the mutant gene may be isolated from progeny of the mutant strand, inserted into a appropriate expression vector, and the vector used to transform a suitable host organism or cell. Preferred vectors are plasmids pBR322, pCR1, and variants thereof, synthetic vectors and the like. It must be recognized that when a host of choice is transformed with the vector, appropriate promoter-operator sequences are also introduced in order for the mutein to be expressed. Hosts may be prokaryotic or eukaryotic (processes for inserting DNA into eukaryotic cells are described in PCT applications nos. US81/00239 and US81/00240 published 3 September 1981). *E. coli* and CHO cells are the preferred hosts. The muteins obtained in accordance with the present invention may be glycosylated or unglycosylated depending on the glycosylation occurring in the native parent protein and the host organism used to produce the mutein. If desired, unglycosylated mutein obtained when *E. coli* is the host organism, may be optionally glycosylated *in vitro* by chemical, enzymatic and other types of modifications known in the art.

The following Examples are presented to help in the better understanding of the subject invention and for purposes of illustration only. They are not to be construed as limiting the scope of the invention.

Example 1

The nucleotide sequence for a cDNA clone coding for human IL-2, procedures for preparing IL-2 cDNA libraries, and screening same for IL-2 are described by Taniguchi, T., et al, *Nature* (1983) *Vol 302*, p 305 et seq.

cDNA libraries enriched in potential IL-2 cDNA clones were made from an IL-2 enriched mRNA fractions obtained from induced peripheral blood lymphocytes (PBL) and Jurkat cells by conventional procedures. The enrichment of the mRNA for IL-2 message was made by fractionating the mRNA and identifying the fraction having IL-2 mRNA activity by injecting the fractions in *Xenopus laevis* oocytes and assaying the oocyte lysates for IL-2 activity on HT-2 cells (J. Watson, *J Exp Med* (1979) *150*:1570—1519 and S. Gillis et al. *J Immun* (1978) *120*:2027—2032.)

Example 2

Screening and identification of IL-2 cDNA clones

The IL-2 cDNA libraries were screened using the colony hybridization procedure. Each microtiter plate was replicated onto duplicate nitrocellulose filter papers (S & S type BA-85) and colonies were allowed to grow at 37°C for 14—16 hr on L agar containing 50 µg/ml ampicillin. The colonies were lysed and DNA fixed to the filter by sequential treatment for 5 min with 500 mM NaOH, 1.5 M NaCl, washed twice for 5 min each time with 5×standard saline citrate (SSC). Filters were air dried and baked at 80°C for 2 hr. The duplicate filters were pre-hybridized at 42°C for 6—8 hr with 10 ml per filter of DNA hybdrization buffer (50% formamide, 5×SSC, pH 7.0, 5×Denhardt's solution (polyvinylpyrrolidine, plus ficoll and bovine serum albumin; 1×=0.2% of each), 50 mM sodium phosphate buffer at pH 7.0, 0.2% SDS, 20 µg/ml Poly U, and 50 µg/ml denatured salmon sperm DNA.

A $^{32}$p-labeled 20-mer oligonucleotide probe was prepared based on the IL-2 gene sequence reported by Taniguchi, T., et al, supra. The nucleotide sequence of the probe was GTGGCCTTCTTGGGCATGTA.

The samples were hybridized at 42°C for 24—36 hr with 5 ml/filter of DNA hybridization buffer containing the $^{32}$p cDNA probe. The filters were washed two times for 30 min each time at 50°C with 2×SSC, 0.1% SDS, then washed twice with 1×SSC and 0.1% SDS at 50°C for 90 min, air dried, and autoradiographed at −70°C for 2 to 3 days. Positive clones were identified and rescreened with the probe. Full length clones were identified and confirmed by restriction enzyme mapping and comparison with the sequence of the IL-2 cDNA clone reported by Taniguchi, T., et al, supra.

Example 3

Cloning of the IL-2 gene into M13 vector:

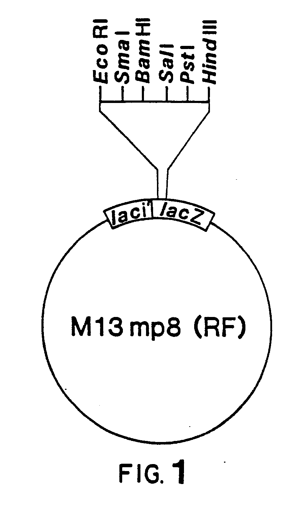

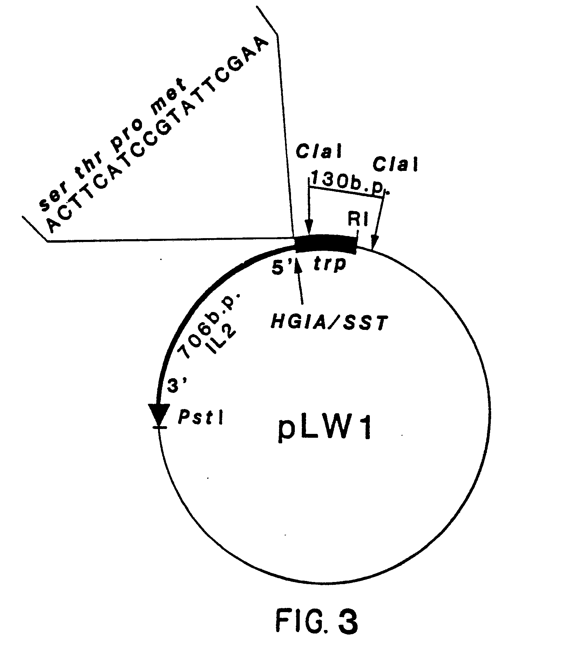

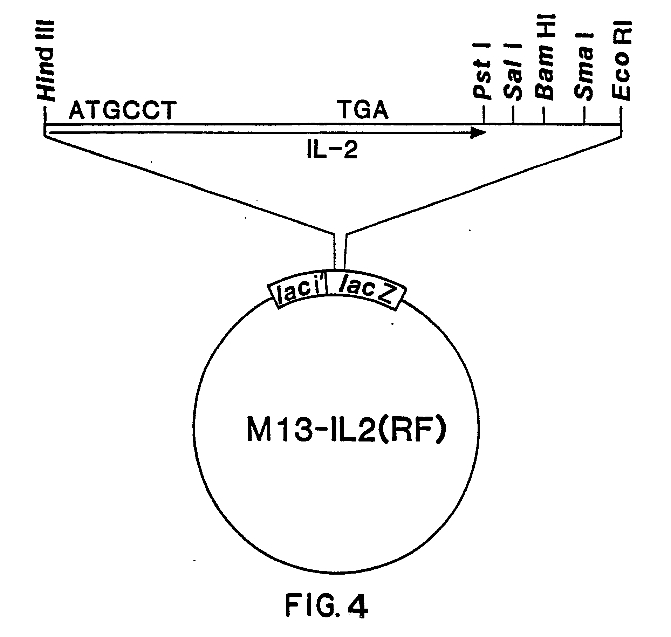

The use of M13 phage vector as a source of single-stranded DNA template has been demonstrated by G. F. Temple et al. *Nature* (1982) *296*:537—540. Plasmid pLW1 (Figure 3) containing the IL-2 gene under control of *E. coli* trp promoter, was digested with the restriction enzymes *Hind*III and *Pst*I. A sample of pLW1 was deposited in the American Type Culture Collection, 12301 Parklawn Drive. Rockville, Maryland 20852, USA. on 4 August 1983 and has been assigned ATCC number 39,405. The M13mp8 (J. Messing, "Third Cleveland Symposium on Macromolecules: Recombinant DNA." Ed. A. Walton, Elsevier Press, 143—153 (1981)) replicative form (RF) DNA (Figure 1) was digested with restriction enzymes *Hind*III and *Bam*HI, and mixed with the pLW1 DNA which had previously been digested with *Hind*III and *Ps*TI. The mixture was then ligated with $T_4$ DNA ligase and the ligated DNA transformed into competent cells of *E. coli* strain JM 103 and plated on Xgal indicator plates (J. Messing, et al. *Nucleic Acids Res* (1981) *9*:309—321). Plaques containing recombinant phage (white plaques) were picked, inoculated into a fresh culture of JM 103 and minipreps of RF molecules prepared from the infected cells (H. D. Birnboim and J. Doly. *Nucleic Acid Res* (1979) *7*:1513—1523). The RF molecules were digested with various restriction enzymes to identify the clones containing the IL-2 insert. The restriction map of one clone (designated M13—IL2) containing the IL-2 insert is shown in Figure 4. Single-stranded (ss) phage DNA was prepared from clone M13—IL2 to serve as a template for site-specific mutagenesis using a synthetic oligonucleotide.

Example 4

Oligonucleotide-directed Mutagenesis

As indicated previously, IL-2 contains cysteine residues at amino acid positions 58, 105 and 125. Based on the nucleotide sequences of the portions of the IL-2 gene that contain the codons for these three cysteine residues three oligonucleotide primers were designed and synthesized for mutating the codons for these residues to codons for serine. These oligonucleotides have the following sequences.

CTTCTAGAGACTGCAGATGTTTC (DM27) to change cys 58,
CATCAGCATACTCAGACATGAATG (DM28) to change cys 105 and
GATGATGCTCTGAGAAAAGGTAATC (DM29) to change cys 125.

Forty picomoles of each oligonucleotide were kinased seperately in the presence of 0.1 mM ATP, 50 mM Tris-HCl, pH 8.0, 10 mM $MgCl_2$, 5 mM DTT and 9 units of $T_4$ kinase in 50 µl at 37°C for 1 hr. Each of the kinased primers (10 pmoles) was hybridized to 2.6 µg of ss M13—IL2 DNA in 15 µl of a mixture containing 100 mM NaCl, 20 mM Tris-HCl, pH 7.9, 20 mM $MgCl_2$ and 20 mM β-mercaptoethanol, by heating at 67°C for 5 min and 42°C for 25 min. The annealed mixtures were chilled on ice and then adjusted to a final column of 25 µl of a reaction mixture containing 0.5 mM of each dNTP, 17 mM Tris-HCl, pH 7.9, 17 mM $MgCl_2$, 83 mM NaCl, 17 mM β-mercaptoethanol, 5 units of DNA polymerase I Klenow fragment, 0.5 mM ATP and 2 units of $T_4$ DNA ligase, incubated at 37°C for 5 hr. The reactions were terminated by heating to 80°C and the reaction mixtures used to transform competent JM103 cells, plated onto agar plates and incubated overnight to obtain phage plaques.

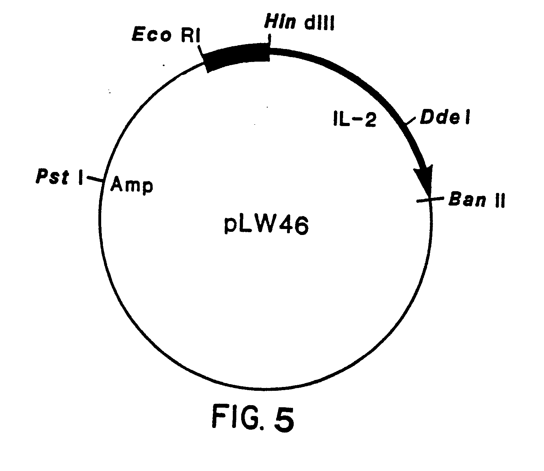

Example 5
Screening and identification of mutagenized phage plaques
Plates containing mutagenized M13—IL2 plaques as well as 2 plates containing unmutagenized M13—IL2 phage plaques, were chilled to 4°C and phage plaques from each plate were transferred onto two nitrocellulose filter circles by layering a dry filter on the agar plate for 5 min for the first filter and 15 min for the second filter. The filters were then placed on thick filter papers soaked in 0.2 N NaOH, 1.5 M NaCl and 0.2% Triton for 5 min, and neutralized by layering onto filter papers soaked with 0.5 M Tris-HCl, pH 7.5, and 1.5 M NaCl for another 5 min. The filters were washed in a similar fashion twice on filters soaked in 2×SSC, dried and then baked in a vacuum oven at 80°C for 2 hr. The duplicate filters were pre-hybridized at 42°C for 4 hr with 10 ml per filter of DNA hybridization buffer (5×SSC, pH 7.0, 4×Denhardts solution (polyvinylpyrrolidine, ficoll and bovin serum albumin, 1×=0.02% of each), 0.1% SDS, 50 mM sodium phosphate buffer, pH 7.0 and 100 µg/ml of denatured salmon sperm DNA. $^{32}$p-labelled probes were prepared by kinasing the oligonucleotide primers with labelled ATP. The filters were hybridized to $0.1 \times 10^5$ cpm/ml of $^{32}$p-labelled primers in 5 ml per filter of DNA hybridization buffer at 42°C for 8 hr. The filters were washed twice at 50°C for 30 min each in washing buffers containing 0.1% SDS and 2×SSC, and twice at 50°C for 30 min each with 0.1% SDS and 0.2×SSC. The filters were air dried and autoradiographed at −70°C for 2—3 days.
Since the oligonucleotide primers DM28 and DM29 were designed to create a new *Dde*I restriction site in the mutagenized clones (Figure 5), RF-DNA from a number of the clones which hybridized with each of these kinased primers were digested with the restriction enzyme *Dde*I. One of the mutagenized M13—IL2 plaques which hybridized with the primer DM28 and has a new *Dde*I restriction site (M13—LW44) was picked and inoculated into a culture of JM103, ssDNA was prepared from the culture supernatant and dsRF-DNA was prepared from the cell pellet. Similarly, a plaque which hybridized with primer DM29 was picked (M13—LW46) and ssDNA and RF-DNA prepared from it. The oligonucleotide primer DM27 was designed to create a new *Pst*I restriction site instead of a *Dde*I site. Therefore, the plaques that hybridized to this primer were screened for the presence of a new *Pst*I site. One such phage plaque was identified (M13—LW42) and ssDNA and RF-DNA prepared from it. The DNA from all three of these clones were sequenced to confirm that the target TGT codons for cysteine had been converted to a TCT codon for serine.

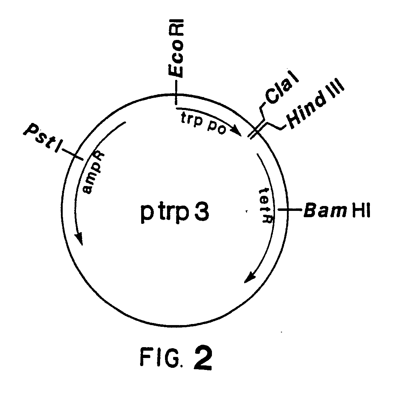

Example 6
Recloning of the mutagenized IL-2 gene for expression in *E. coli*
RF-DNA from M13—LW42, M13—LW44 and M13—LW46 were each digested with restriction enzymes *Hind*III and *Ban*II and the insert fragments purified from a 1% agarose gel. Similarly, the plasmid pTrp3 (Figure 2) was digested with *Hind*III and *Ban*II, the large plasmid fragment containing the trp promoter was purified on an agarose gel and then ligated with each of the insert fragments isolated from M13—LW42, M13—LW44 and M13—LW46. The ligated plasmids were transformed into competent *E. coli* K12 strain MM294. The plasmid DNAs from these transformants were analysed by restriction enzyme mapping to confirm the presence of the plasmids pLW42, pLW44 and pLW46. Figure 5 is a restriction map of pLW46. When each of these individual clones were grown in the absence of tryptophan to induce the trp promoter and cell free extracts analyzed on SDS-polyacrylamide gels, all three clones, pLW42, pLW44 and pLW46, were shown to synthesize a 14.5 kd protein similar to that found in the positive control, pLW21, which has been demonstrated to synthesize a 14.4 kd IL-2 protein. When these same extracts were subjected to assay for IL-2 activity on mouse HT-2 cells, only clones pLW21 (positive control) and pLW46 had significant amounts of IL-2 activity (Table below), indicating that cys 58 and cys 105 are necessary for biological activity and changing them to serines (pLW42 and pLW44 respectively) resulted in the loss of biological activity. Cys 125 on the other hand must not be necessary for biological activity because changing it to ser 125 (pLW46) did not affect the biological activity.

TABLE

| Clones | IL-2 activity (µ/ml) |
|---|---|
| pIL2—7 (negative control) | 1 |
| pLW21 (positive control) | 113,000 |
| pLW42 | 660 |
| pLW44 | 1,990 |
| pLW46 | 123,000 |

Figure 6a shows the nucleotide sequence of the coding strand of clone pLW46. As compared to the coding strand of the native human IL-2 gene clone pLW46 has a single base change of G→C at nucleotide 374. Figure 6b shows the corresponding amino acid sequence of the IL-2 mutein encoded by pLW46. This mutein it designated IL-2$_{ser125}$. As compared to native IL-2 the mutein has a serine instead of a cysteine at position 125.

A sample of E. coli K12 strain MM294 transformed with pLW46 was deposited in the American Type Culture Collection, 12301 Parklawn Drive, Rockville, Maryland 20852, USA on 26 September 1983 and has been assigned ATCC Number 39,452.

Muteins of IL-2 in whcih the cysteine at position 125 has been replaced with another amino acid, such as the mutein IL-2$_{ser125}$ retain IL-2 activity. They may, therefore, be formulated and used in the same manner as native IL-2. Accordingly, such IL-2 muteins are useful in the diagnosis and treatment of bacterial, viral, parasitic, protozoan and fungal infections; in manifestations of lymphokine or immunodeficiency; for reconstitution of normal immunofunction in aged humans and animals; and in various other therapeutic, diagnostic and research applications. The various therapeutic and diagnostic applications of human IL-2 have been investigated and reported in S. A. Rosenberg, E. A. Grimm, et al, A. Mazumder, et al, and E. A. Grimm and S. A. Rosenberg. IL-2 muteins may be used by themselves or in combination with other immunologically relevant B or T cells or other therapeutic agents. For therapeutic or diagnostic applications (whether for human or veterinary use), they may, for example, be formulated in non-toxic, nonallergenic, physiologically compatible carrier media such as distilled water, Ringer's solution, Hank's solution, physiological saline and the like. Administration of the IL-2 muteins to humans or animals may be oral or intraperitoneal or intramuscular or subcutaneous as deemed appropriate by the physician. Examples of relevant cells are B or T cells, natural killer cells, and the like and exemplary therapeutic reagents which may be used in combination with the polypeptides of this invention are the various interferons, especially gamma interferon, B cell growth factor, IL-1 and the like.

**Claims**

1. A biologically active recombinant human interleukin-2 mutein pharmaceutical or veterinary preparation suitable for use in therapeutic or diagnostic applications, which preparation is incapable of forming an improper intramolecular disulfide bridge and is stable upon storage relative to bacterially derived reduced native interleukin-2, wherein the cysteine residue normally present at position 125, numbered in accordance with native interleukin-2, that is free to form a disulfide link and is non-essential to said biological activity has been substituted by a neutral amino acid.

2. A mutein preparation as claimed in claim 1 which is a serine$_{125}$ interleukin-2 mutein preparation, a threonine$_{125}$interleukin-2 mutein preparation, a glycine$_{125}$interleukin-2 mutein preparation, an alanine$_{125}$interleukin-2 mutein preparation, a valine$_{125}$interleukin-2 mutein preparation, a leucine$_{125}$interleukin-2 mutein preparation, an isoleucine$_{125}$interleukin-2 mutein preparation, a histidine$_{125}$interleukin-2 mutein preparation, a tyrosine$_{125}$interleukin-2 mutein preparation, a phenylalanine$_{125}$interleukin-2 mutein preparation, a tryptophan$_{125}$interleukin-2 mutein preparation, or a methionine$_{125}$interleukin-2 mutein preparation.

3. A mutein preparation as claimed in claim 1, wherein said neutral amino acid is serine.

4. A human recombinant serine$_{125}$interleukin-2 mutein pharmaceutical or veterinary preparation suitable for use in therapeutic or diagnostic applications which is incapable of forming an improper intramolecular disulfide bridge and is stable upon storage relative to bacterially derived reduced native interleukin-2.

5. A human recombinant serine$_{125}$interleukin-2 mutein preparation which exhibits the biological activity of native human interleukin-2 and which is incapable of forming an improper intramolecular disulfide bridge but which is stable upon storage relative to bacterially derived reduced native interleukin-2, and which has the deduced amino acid sequence shown in Figure 6b.

6. A mutein preparation as claimed in any one of claims 1 to 5 and which is unglycosylated.

7. A synthetic gene which has a DNA sequence encoding a human interleukin-2 polypeptide having an amino acid sequence as shown in Fig. 6b, optionally in which the serine residue in position 125, numbered in accordance with native interleukin-2, is an alternative neutral amino acid other than cysteine.

8. An expression vector that includes a gene as defined in claim 7.

9. Plasmid pLW46, as obtainable from the E. coli strain deposited under the accession nr. ATCC 39452.

10. *E. coli* transformed with an expression vector as defined in claim 8 or with a plasmid as defined in claim 9.

11. The use of an expression vector as defined in claim 8 or a plasmid as defined in claim 9 in making a recombinant interleukin-2 mutein preparation by expressing said vector or plasmid.

12. A method of preventing an interleukin-2 protein from forming an improper cysteine-derived disulfide link and for stabilizing said protein upon storage relative to bacterially derived reduced native interleukin-2, which method comprises mutationally altering the protein by substituting the cysteine residue at position 125, numbered in accordance with native human interleukin-2, with a neutral amino acid.

13. A method as claimed in claim 12, wherein the cysteine residue is substituted with serine or threonine.

14. A method for making a synthetic gene as defined in claim 7 comprising:

(a) hybridizing a single-stranded DNA comprising a strand of a gene that encodes for an interleukin-2 protein with a mutant oligonucleotide primer that is complementary to a region of said strand that includes the codon for said cysteine residue or the antisense triplet paired with said codon, as the case may be, except for a mismatch with said codon or said antisense triplet which mismatch defines a triplet that codes for said neutral amino acid;

(b) extending·the primer with DNA polymerase to form a mutational heteroduplex; and

(c) replicating said mutational heteroduplex.

15. A method as claimed in claim 14, wherein the mismatch defines a triplet that codes for serine or threonine.

16. A method as claimed in claim 14, or claim 15, wherein the single-stranded DNA is a single-stranded phage that includes said strand and the mutational heteroduplex of step (b) is converted to a closed circular heteroduplex.

17. A method as claimed in any one of claims 14 to 16, wherein said replicating is effected by transforming a competent bacterial host with the closed circular heteroduplex and culturing the resulting transformants.

18. A method as claimed in any one of claims 14 to ·17 and including the additional steps of isolating progeny of the mutant strand of the heteroduplex, isolating DNA from said progeny, and isolating said gene from the DNA from said progeny.

19. A method as claimed in any one of claims 14 to 18, wherein the protein is human interleukin-2, the cysteine residue is at position 125 and the mismatch defines a codon that codes for serine.

20. A oligonucleotide for use in making a synthetic gene as defined in claim 7 by oligonucleotide-directed mutagenesis and having a nucleotide sequence that is complementary to a region of the strand of the gene that includes the codon for the cysteine residue or the antisense triplet paired with said codon, as the case may be, except for a mismatch with said codon that defines a triplet that codes for said neutral amino acid.

21. A process for preparing an oligonucleotide suitable for use in making a synthetic gene as defined in claim 7 by oligonucleotide-directed mutagenesis which comprises synthesizing by a manner known per se a nucleotide sequence that is complementary to a region of the strand of the gene that includes the codon for the cysteine residue or the antisense triplet paired with said codon, as the case may be, except for a mismatch with said codon that defines a triplet that codes for a neutral amino acid.

22. A process for producing a vector as defined in claim 8 comprising inserting into a vector capable of transforming a host cell a gene as defined in claim 7 in a position permitting expression of said gene.

23. A pharmaceutical or veterinary formulation having interleukin-2 activity and comprising a biologically active recombinant human interleukin-2 mutein, wherein the cysteine residue normally present at position 125, numbered in accordance with native interleukin-2, has been substituted by a neutral amino acid, formulated for pharmaceutical or veterinary use, respectively, and optionally also comprising a pharmceutically or veterinarily acceptable diluent, carrier or excipient and/or optionally being in unit dosage form.

24. A formulation for use in either: the diagnosis or therapeutic treatment of bacterial, viral, parasitic, protozoan and fungal infections; manifestations of lymphokine or immuno-deficiency; the reconstitution of immunofunction in aged humans and animals: comprising:

(a) an effective amount of recombinant human interleukin-2 mutein, wherein the cysteine residue at position 125, numbered in accordance with native human interleukin-2, is substituted by a neutral amino acid and said mutein exhibits the biological activity of native human interleukin-2; and

(b) a non-toxic, non-allergenic, therapeutically acceptable carrier medium.

25. A formulation as claimed in claim 24, wherein the mutein is a serine$_{125}$-interleukin-2 and said carrier is distilled water, Ringer's solution, Hank's solution or physiological saline.

26. A formulation comprising:

(a) a recombinant human interleukin-2 mutein, wherein the cysteine residue at position 125, numbered in accordance with native human interleukin-2, is substituted by a neutral amino acid and said mutein exhibits the biological activity of native, human interleukin-2; and

(b) a polypeptide selected from gamma interferon, B cell growth factor and IL-1.

7

27. A formulation as claimed in claim 26, wherein the mutein is a serine₁₂₅interleukin-2.

28. A pharmaceutical or veterinary formulation which is stable against inactivation upon storage and which comprises, formulated for pharmaceutical or veterinary use, respectively, a biologically active recombinant human interleukin-2 mutein wherein the cysteine residue normally present at position 125, numbered in accordance with native interleukin-2, has been substituted by a neutral amino acid.

29. The use of a biologically active recombinant human interleukin-2 mutein, wherein the cysteine residue normally present at position 125, numbered in accordance with native interleukin-2, has been substituted by a neutral amino acid, in preparing a pharmaceutical or veterinary formulation having interleukin-2 activity.

30. The use of directed mutagenesis techniques to produce a DNA sequence which encodes a mutein of human interleukin-2, wherein the cysteine residue normally present at position 125, numbered in accordance with native interleukin-2, has been substituted by a neutral amino acid.

31. The use, in the preparation of a DNA sequence encoding a mutein of human interleukin-2, in which the cysteine residue normally present as position 125, numbered in accordance with native interleukin-2, has been substituted by a neutral amino acid, of native human interleukin-2 cDNA.

32. A mutein preparation as defined in any one of claims 1 to 6 for use in prophylaxis, therapy or diagnosis practised on the human or animal body.

33. A method for making a pharmaceutical or veterinary formulation comprising formulating for pharmaceutical or veterinary use, respectively, a mutein preparation as defined in any one of claims 1 to 6.

34. A method for stabilizing a human interleukin-2 protein comprising substituting the cysteine residue at position 125, numbered in accordance with native human interleukin-2, with a neutral amino acid.

35. A human recombinant serine₁₂₅interleukin-2 mutein which is incapable of forming an improper intramolecular disulfide bridge but which is stable upon storage relative to bacterially derived reduced native interleukin-2, and which has the deduced amino acid sequence shown in Figure 6b.

36. A pharmaceutical or veterinary formulation comprising a mutein as claimed in claim 35 formulated for pharmaceutical or veterinary use, respectively.

37. The use of a mutein as claimed in claim 35 in making a medicament.

**Patentansprüche**

1. Ein pharmazeutisches oder tiermedizinisches, zur Verwendung in Therapie oder Diagnose geeignetes biologisch aktives, rekombinantes, menschliches Interleukin-2-Muteinpräparat, das keine untaugliche intramolekulare Disulfidbrücke bilden kann und verglichen mit von Bakterien abgeleitetem, reduziertem, natürlichem Interleukin-2 bei der Lagerung stabil ist, und in dem der normalerweise in Position 125, entsprechend der Numerierung von natürlichem Interleukin-2, befindliche Cysteinrest, der zur Bildung einer Disulfidbrücke zur Verfügung steht und unwichtig für die biologische Aktivität ist, durch eine neutrale Aminosäure substituiert ist.

2. Muteinpräparat nach Anspruch 1, das ein Serin₁₂₅-Interleukin-2-Muteinpräparat, ein Threonin₁₂₅-Interleukin-2-Muteinpräparat, ein Glycin₁₂₅-Interleukin-2-Muteinpräparat, ein Alanin₁₂₅-Interleukin-2-Muteinpräparat, ein Valin₁₂₅-Interleukin-2-Muteinpräparat, ein Leucin₁₂₅-Interleukin-2-Muteinpräparat, ein Isoleucin₁₂₅-Interleukin-2-Muteinpräparat, ein Histidin₁₂₅-Interleukin-2-Muteinpräparat, ein Tyrosin₁₂₅-Interleukin-2-Muteinpräparat, ein Phenylalanin₁₂₅-Interleukin-2-Muteinpräparat, ein Tryptophan₁₂₅-Interleukin-2-Muteinpräparat oder ein Methionin₁₂₅-Interleukin-2-Muteinpräparat ist.

3. Muteinpräparat nach Anspruch 1, in dem die neutrale Aminosäure Serin ist.

4. Pharmazeutisches oder tiermedizinisches, zur Verwendung in Therapie oder Diagnose geeignetes rekombinantes, menschliches Serin₁₂₅-Interleukin-2-Muteinpräparat, das keine untaugliche intramolekulare Disulfidbrücke bilden kann und das verglichen mit von Bakterien abgeleitetem, reduziertem, natürlichem Interleukin-2 bei der Lägerung stabil ist.

5. Menschliches, rekombinantes Serin₁₂₅-Interleukin-2-Muteinpräparat, das die biologische Aktivität von natürlichem, menschlichem Interleukin-2 hat und keine untaugliche Disulfidbrücke bilden kann, jedoch verglichen mit von Bakterien abgeleitetem, reduziertem, natürlichem Interleukin-2 bei der Lagerung stabil ist, und die abgeleitete, in Figur 6b gezeigte Aminosäure-Sequenz aufweist.

6. Muteinpräparat nach einem der Ansprüche 1 bis 5, das nicht glykosiliert ist.

7. Synthetisches Gen, das eine DNA-Sequenz aufweist, die ein menschliches Interleukin-2-Polypeptid mit einer wie in Figur 6b gezeigten Aminosäure-Sequenz codiert, in der gegebenenfalls der an Position 125, entsprechend der Numerierung von näturlichem Interleukin-2, befindliche Serinrest eine andere neutrale Aminosäure, jedoch nicht Cystein ist.

8. Expressionsvektor, der ein Gen nach Anspruch 7 aufweist.

9. Plasmid pLW46, das aus dem unter der Hinterlegungs-Nummer ATCC 39452 hinterlegten E. coli-Stamm erhältlich ist.

10. *E. coli*, das mit einem Expressionsvektor nach Anspruch 8 oder mit einem Plasmid nach Anspruch 9 transformiert ist.

11. Verwendung eines Expressionsvektors nach Anspruch 8 oder eines Plasmids nach Anspruch 9 zur Herstellung eines rekombinanten Interleukin-2-Muteinpräparats durch Expression des Vektors oder des Plasmids.

12. Verfahren zur Verhinderung der Bildung einer untauglichen Cystein-abgeleiteten Disulfidbrücke durch ein Interleukin-2-Protein und zur Stabilisierung des Proteins bei der Lagerung, verglichen mit von Bakterien abgeleitetem, reduziertem, natürlichem Interleukin-2, bei dem das Protein durch Mutation geändert wird, indem der Cysteinrest an Position 125, numeriert entsprechend natürlichem, menschlichem Interleukin-2, durch eine neutrale Aminosäure ersetzt wird.

13. Verfahren nach Anspruch 12, bei dem der Cysteinrest durch einen Serin- oder einen Threoninrest ersetzt wird.

14. Verfahren zur Herstellung eines synthetischen Gens nach Anspruch 7, das umfaßt:

(a) Hybridisierung einer Hinzelstrang-DNA, die einen Strang eines Interleukin-2-Protein-codierenden Gens enthält, mit einem mutierten Oligonucleotidprimer, der komplementär zu einem Bereich des Stranges ist, der fallweise das Codon für den Cysteinrest oder das Gegenstrang-Triplett gepaart mit dem Codon, enthält, mit Ausnahme einer Fehlpaarung mit dem Codon oder dem Gegenstrang-Triplett, durch die ein Triplett bestimmt wird, das die neutrale Aminosäure codiert;

(b) Verlängerung des Primers mit DNA-Polymerase unter Bildung einer mutierten Heteroduplex-Struktur; und

(c) Replikation der mutierten Heteroduplex-Struktur.

15. Verfahren nach Anspruch 14, bei dem durch die Fehlpaarung ein für Serin oder Threonin codierendes Triplett bestimmt wird.

16. Verfahren nach Anspruch 14 oder 15, bei dem die Einzelstrang-DNA ein einzelsträngiger, den Strang enthaltender Phage ist und die mutierte Heteroduplex-Struktur aus Schritt (b) in eine geschlossene, zirkoläre Heteroduplex-Struktur überführt wird.

17. Verfahren nach einem der Ansprüche 14 bis 16, bei dem die Replikation durch Transformation eines kompetenten bakteriellen Wirtes mit der geschlossenen, zirkulären Heteroduplex-Struktur und durch Züchtung der so erhaltenen Transformanten erreicht wird.

18. Verfahren nach einem der Ansprüche 14 bis 17, bei dem außerdem die Nachkommen des mutierten Stranges der Heteroduplex-Struktur isoliert werden, DNA aus den Nachkommen isoliert und aus der DNA der Nachkommen das Gen isoliert wird.

19. Verfahren nach einem der Ansprüche 14 bis 18, bei dem das Protein menschliches Interleukin-2 ist, sich an Position 125 ein Cysteinrest befindet und durch die Fehlpaarung ein Coden bestimmt wird, das für Serin codiert.

20. Oligonucleotid zur Verwendung bei der Herstellung eines synthetischen Gens nach Anspruch 7 durch Oligonucleotid-gesteuerte Mutagenese mit einer Nucleotidsequenz, die zu einem Bereich des Stranges des Gens komplementär ist, der fallweise das Codon für den Cysteinrest oder das Gegenstrang-Triplett gepaart mit dem Codon enthält, mit Ausnahme einer Fehlpaarung mit dem Codon, durch die ein Triplett bestimmt wird, das für die neutrale Aminosäure codiert.

21. Verfahren zur Herstellung eines zur Verwendung bei der Herstellung eines synthetischen Gens nach Anspruch 7 durch Oligonucleotid-gesteuerte Mutagenese geeigneten Oligonucleotids, durch Synthetisieren in an sich bekannter Weise eine Nucleotidsequenz, die zu einem Bereich des Stranges des Gens komplementär ist, der fallweise das Codon für den Cysteinrest oder das Gegenstrang-Triplett gepaart mit dem Codon enthält, mit Ausnahme einer Fehlpaarung mit dem Codon, durch die Triplett bestimmt wird, das für eine neutrale Aminosäure codiert.

22. Verfahren zur Herstellung eines Vektors nach Anspruch 8 durch Inserieren eines Gens nach Anspruch 7 in einen zur Transformation einer Wirtszelle geeigneten Vektor an einer Stelle, die die Expression des Gens gestattet.

23. Arzneimittel oder tiermedizinisches Mittel mit Interleukin-2-Aktivität, enthaltend ein biologisch aktives, rekombinantes, menschliches Interleukin-2-Mutein, in dem der normalerweise an Position 125, entsprechend der Numerierung von natürlichem Interleukin-2, befindliche Cysteinrest durch eine neutrale Aminosäure substituiert ist, das zur pharmazeutischen bzw. tiermedizinischen Verwendung formuliert ist, und gegebenenfalls zusätzlich ein pharmazeutisch oder tiermedizinisch verträgliches Verdünnungsmittel, Träger oder Exzipienz enthält und/oder gegebenenfalls als Einheitsdosierung vorliegt.

24. Mittel zur Verwendung bei der Diagnose oder therapeutischen Behandlung bakterieller, viraler, parasitischer, protozoischer und pilzbedingter Infektionen; bei Symptomen von Lymphokine oder Immundefizienz; oder bei der Wiederherstellung der Funktion des Immunsystems bei alten Menschen und Tieren; enthaltend:

(a) eine wirksame Menge eines rekombinanten, menschlichen Interleukin-2-Muteins, in dem der Cysteinrest an Position 125, entsprechend der Numerierung von natürlichem, menschlichem Interleukin-2, durch eine neutrale Aminosäure substituiert ist, und das die biologische Aktivität von natürlichem, menschlichem Interleukin-2-hat; und

(b) einen nicht-toxischen, nicht-allergenen, therapeutisch verträglichen Träger.

25. Mittel nach Anspruch 24, in dem das Mutein ein $Serin_{125}$-Interleukin-2 und der Träger destilliertes Wasser, Ringer'sche Lösung, Hank'sche Lösung oder physiologische Kochsalzlösung ist.

26. Mittel enthaltend:

(a) rekombinantes, menschliches Interleukin-2-Mutein, in dem der Cysteinrest an Position 125, entsprechend der Numerierung von natürlichem, menschlichem Interleukin-2, durch eine neutrale Aminosäure substituiert ist, und das die biologische Aktivität von natürlichem, menschlichem Interleukin-2 hat; und

(b) eines der Polypeptide Gamma-Interferon, B-Zellen-Wachstumsfaktor oder IL-1.

27. Mittel nach Anspruch 26, in dem das Mutein ein Serin$_{125}$-Interleukin-2 ist.

28. Arzneimittel oder tiermedizinisches Mittel, das stabil gegen Inaktivierung durch Lagerung ist und das, formuliert zur pharmazeutischen bzw. tiermedizinischen Verwendung, ein biologisch aktives, rekombinantes, menschliches Interleukin-2-Mutein enthält, in dem der normalerweise an Position 125, entsprechend der Numerierung von natürlichem Interleukin-2, befindliche Cysteinrest durch eine neutrale Aminosäure substituiert ist.

29. Verwendung eines biologisch aktiven, rekombinanten, menschlichen Interleukin-2-Muteins, in dem der normalerweise an Position 125, entsprechend der Numerierung von natürlichem Interleukin-2, befindliche Cysteinrest durch eine neutrale Aminosäure substituiert ist, zur Herstellung eines Arzneimittels oder eines tiermedizinischen Mittels mit Interleukin-2-Aktivität.

30. Verwendung von gezielten Mutagenese-Techniken zur Herstellung einer DNA-Sequenz, die ein Mutein des menschlichen Interleukin-2 codiert, in dem der normalerweise an Position 125, entsprechend der Numerierung von natürlichem Interleukin-2, befindliche Cysteinrest durch eine neutrale Aminosäure substituiert ist.

31. Verwendung natürlicher, menschlicher Interleukin-2-cDNA zur Herstellung einer DNA-Sequenz, die ein Mutein des menschlichen Interleukin-2 codiert, in dem der normalerweise an Position 125, entsprechend der Numerierung von natürlichem Interleukin-2, befindliche Cysteinrest durch eine neutrale Aminosäure substituiert ist.

32. Muteinpräparat nach einem der Ansprüche 1 bis 6 zur Verwendung in Prophylaxe, Therapie oder Diagnose am Körper von Mesch oder Tier.

33. Verfahren zur Herstellung eines Arzneimittels oder eines tiermedizinischen Mittels, durch Formulierung eines Muteinpräparates nach einem der Ansprüche 1 bis 6 zur pharmazeutischen oder tiermedizinischen Verwendung.

34. Verfahren zur Stabilisierung eines menschlichen Interleukin-2-Proteins, durch Ersetzen des Cysteinrestes an Position 125, entsprechend der Numerierung von natürlichem, menschlichem Interleukin-2, durch eine neutrale Aminosäure.

35. Menschliches, rekombinantes Serin$_{125}$-Interleukin-2-Mutein, das keine untaugliche intramolekulare Disulfidbrücke bilden kann, jedoch verglichen mit von Bakterien abgeleitetem, reduziertem, natürlichem Interleukin-2 bei der Lagerung stabil ist, und die abgeleitete, in Figur 6b gezeigte Aminosäuresequenz aufweist.

36. Arzneimittel oder tiermedizinisches Mittel, enthaltend ein Mutein nach Anspruch 35, das zur pharmazeutischen oder tiermedizinischen Verwendung formuliert ist.

37. Verwendung eines Muteins nach Anspruch 35 zur Herstellung eines Arzneimittels.

## Revendications

1. Préparation pharmaceutique ou vétérinaire de mutéine d'interleukine-2 humaine recombinante biologiquement active, convenant pour des applications therapeutiques ou de diagnostic, ladite préparation étant incapable de former un pont disulfure intramoléculaire non approprié et étant stable au stockage par rapport à l'interleukine-2 native réduite obtenue par voie bactérienne, le reste cystéine présent normalement en position 125, dans la numérotation correspondant à l'interleukine-2 native, qui est libre de former une liaison disulfure et est non essentiel à ladite activité biologique, ayant été remplacé par un acide aminé neutre.

2. Préparation de mutéine selon la revendication 1, qui est une préparation de sérine-$_{125}$ interleukine-2 mutéine, une préparation de thréonine-$_{125}$ interleukine-2 mutéine, une préparation de glycine-$_{125}$ interleukine-2 mutéine, une préparation d'alanine-$_{125}$ interleukine-2 mutéine, une préparation de valine-$_{125}$ interleukine-2 mutéine, une préparation de leucine-$_{125}$ interleukine-2 mutéine, une préparation d'isoleucine-$_{125}$ interleukine-2 mutéine, une préparation d'histidine-$_{125}$ interleukine-2 mutéine, une préparation de tyrosine-$_{125}$ interleukine-2 mutéine, une préparation de phénylalanine-$_{125}$ interleukine-2 mutéine, une préparation de tryptophane-$_{125}$ interleukine-2 mutéine ou une préparation de méthionine-$_{125}$ interleukine-2 mutéine.

3. Préparation de mutéine selon la revendication 1, dans laquelle l'acide aminé neutre est la sérine.

4. Préparation pharmaceutique ou vétérinaire de sérine-$_{125}$ interleukine-2 mutéine recombinante humaine convenant pour des applications thérapeutiques ou de diagnostic, qui est incapable de former un pont disulfure intra-moléculaire non approprié et est stable au stockage par rapport à l'interleukine-2 native réduite obtenue par voie bactérienne.

5. Préparation de sérine-$_{125}$ interleukine-2 mutéine recombinante humaine qui présente l'activité biologique de l'interleukine-2 humaine native et qui est incapable de former un pont disulfure intra-moléculaire non approprié mais qui est stable au stockage par rapport à l'interleukine-2 native réduite obtenue par voie bactérienne et qui a la séquence d'acides aminés déduite représentée sur la Figure 6b.

6. Préparation de mutéine selon l'une quelconque des revendications 1 à 5 et qui est non glycosylée.

7. Gène synthétique qui a une séquence d'ADN codant pour un polypeptide d'interleukine-2 humaine

10

ayant une séquence d'acides aminés comme représentée sur la Figure 6b, dans laquelle éventuellement le reste sérine en position 125, dans la numérotation correspondant à l'interleukine-2 native, est remplacé par un acide aminé neutre autre que la cystéine.

8. Vecteur d'expression qui comprend un gène tel que défini à la revendication 7.

9. Plasmide pLW46 tel que pouvant être obtenu à partir de la souche de E. Coli déposé sous le numéro ATCC 39452.

10. *E. Coli* transformé avec un vecteur d'expression tel que défini à la revendication 8 ou avec un plasmide tel que défini à la revendication 9.

11. Utilisation d'un vecteur d'expression tel que défini dans la revendication 8 ou d'un plasmide défini dans la revendication 9, dans la fabrication d'une préparation de mutéine d'interleukine-2 recombinante par expression dudit vecteur ou dudit plasmide.

12. Procédé pour empêcher une protéine d'interleukine-2 de former une liaison disulfure non approprié provenant de la cystéine et pour stabiliser la protéine au stockage par rapport à l'interleukine-2 native réduite obtenue par voie bactérienne, ladite méthode comprenant la modification de la protéine par mutation en remplaçant le reste cystéine en position 125, dans la numérotation correspondant à l'interleukine-2 humaine active, par un acide aminé neutre.

13. Procédé selon la revendication 12, dans lequel un reste cystéine est remplacé par la série ou la thréonine.

14. Procédé pour produire un gène synthétique selon la revendication 7 dans lequel

(a) on hybride un ADN mono-caténaire comprenant une chaîne d'un gène qui code pour une protéine d'interleukine-2, avec un primaire oligonucléotide mutant qui est complémentaire d'une région de ladite chaîne qui comprend le codon pour le reste cystéine ou le triplet "anti-sens" apparié audit codon, selon le cas à l'exception d'un mauvais assortiment avec le codon ou le triplet "anti-sens", lequel mauvais assortiment définit un triplet qui code pour ledit acide aminé neutre;

(b) on prolonge le primaire avec l'ADN polymérase pour former un héteroduplex de mutation; et

(c) on réplique l'hétéroduplex de mutation.

15. Procédé selon la revendication 14, dans lequel le mauvais assortiment définit un triplet qui code pour la sérine ou la thréonine.

16. Procédé selon la revendication 14 ou la revendication 15, dans lequel l'ADN mono-caténaire est un phage mono-caténaire qui comprend ladite chaîne et l'hétéroduplex de mutation du stade (b) est converti en un hétéroduplex circulaire fermé.

17. Procédé selon l'une quelconque des revendications 14 à 16, dans lequel la réplication est effectuée en transformant un hôte bactérien compétent avec l'hétéroduplex circulaire fermé et en cultivant des produits de transformation résultants.

18. Procédé selon l'une quelconque des revendications 14 à 17 et comprenant comme autres stades l'isolement de la descendance de la chaîne mutante de l'hétéroduplex, l'isolement de l'ADN de ladite descendance et l'isolement du gène à partir de l'ADN de la descendance.

19. Procédé selon l'une quelconque des revendications 14 à 18, dans lequel la protéine est l'interleukine-2 humaine, le reste cystéine est en position 125 et le mauvais assortiment définit un codon qui code pour la sérine.

20. Oligonucléotide utilisable pour produire un gène synthétique selon la revendication 7 par une mutagénèse dirigée sur un oligonucléotide, et ayant une séquence de nucléotides qui est complémentaire de la région de la chaîne de gène qui comprend le codon pour le reste cystéine ou le triplet "anti-sens" apparié audit codon, selon le cas, à l'exception d'un mauvais assortiment avec le codon, lequel mauvais assortiment définit un triplet qui code pour ledit acide aminé neutre.

21. Procédé de préparation d'un oligonucléotide utilisable pour produire un gène synthétique tel que défini à la revendication 7 par une mutagénèse dirigée sur un oligonucléotide qui comprend la synthèse d'une manière connue en soi d'une séquence de nucléotide qui est complémentaire d'une région de la chaîne du gène qui comprend le codon pour le reste cystéine ou le triplet "anti-sens" apparié audit codon, selon le cas, à l'exception d'un mauvais assortiment avec ledit codon, lequel mauvais assortiment définit un triplet qui code pour un acide aminé neutre.

22. Procédé de production d'un vecteur selon la revendication 8 comprenant l'insertion, dans un vecteur capable de transformer une cellule hôte, d'un gène tel que défini dans la revendication 7 en une position permettant l'expression dudit gène.

23. Formulation pharmaceutique or vétérinaire ayant une activité d'interleukine-2 et comprenant une mutéine d'interleukine-2 humaine recombinante biologiquement active dans laquelle le reste cystéine normalement présent en position 125, dans la numérotation correspondant à l'interleukine-2 native, a été remplacée par un acide aminé neutre, formulée pour une utilisation pharmaceutique ou vétérinaire, respectivement, et eventuellement comprenant également un diluant, véhicule ou excipient acceptable du point de vue pharmaceutique ou vétérinaire et/ou éventuellement sous forme de dose unitaire.

24. Formulation pour une utilisation dans le diagnostic ou le traitement therapeutique des infections provoquées par des bactéries, des virus, des parasites, des protozoaires et des fongis, les manifestations de lymphokine ou d'immuno-déficience ou la reconstitution de la fonction immune chez les hommes et les animaux âgés, comprenant:

(a) une quantité efficace de mutéine d'interleukine-2 humaine recombinante dans laquelle le reste

11

cystéine en position 125, dans la numérotation correspondant à l'interleukine-2 humaine native, est remplacée par un acide aminé neutre et ladite mutéine présente l'activité biologique de l'interleukine-2 humaine native, et

(b) un véhicule non toxique, non allergénique thérapeutiquement acceptable.

25. Formulation selon la revendication 24, dans laquelle la mutéine est une sérine-$_{125}$ interleukine-2 et ledit véhicule est de l'eau distillée, un solution de Ringer, une solution de Hank ou du sérum physiologique.

26. Formulation comprenant

(a) une mutéine d'interleukine-2 humaine recombinante dans laquelle le reste cystéine en position 125, dans la numérotation correspondant à l'interleukine-2 humaine native, est remplacé par un acide aminé neutre et ladite mutéine présente l'activité biologique de l'interleukine-2 humaine native, et

(b) un polypeptide choisi parmi le gamma interféron, le facteur de croissance des cellules B et IL-1.

27. Formulation selon la revendication 26, dans laquelle la mutéine est une sérine-$_{125}$ interleukine-2.

28. Formulation pharmaceutique ou vétérinaire qui est stable vis-à-vis d'une inactivation au stockage et qui comprend, formulée pour une utilisation pharmaceutique ou vétérinaire, respectivement, une mutéine d'interleukine-2 humaine recombinante biologiquement active dans laquelle le reste cystéine normalement présent en position 125, dans la numérotation correspondant à l'interleukine-2 humaine native, à été remplacée par un acide aminé neutre.

29. Utilisation de mutéine d'interleukine-2 humaine recombinante biologiquement active dans laquelle le reste cystéine normalement présent en position 125, dans la numérotation correspondant à l'interleukine-2 humaine native, a été remplacée par un acide aminé neutre, pour la préparation d'une formulation pharmaceutique ou vétérinaire ayant une activité interleukine-2.

30. Utilisation de technique de mutagénèse dirigée pour produire une séquence d'ADN qui code pour mutéine d'interleukine-2 humaine dans laquelle le reste cystéine normalement présent en position 125, dans la numérotation correspondant à l'interleukine-2 native, a été remplacée par un acide aminé neutre.

31. Utilisation dans la préparation d'une séquence d'ADN codant pour une mutéine d'interleukine-2 humaine, dans laquelle le reste cystéine normalement présent en position 125, dans la numérotation correspondant à l'interleukine-2 native, a été remplacée par un acide aminé neutre, d'ADNc d'interleukine-2 humaine native.

32. Préparation de mutéine selon l'une quelconque des revendications 1 à 6, pour une utilisation dans la prophylaxie, la thérapie, ou le diagnostic pratiqué sur le corps humain ou animal.

33. Un procédé de fabrication d'une formulation pharmaceutique ou vétérinaire comprenant la mise sous une forme convenant pour une utilisation pharmaceutique ou vétérinaire, respectivement, d'une préparation à base de mutéine telle que définie dans les revendications 1 à 6.

34. Procédé pour stabiliser une protéine d'interleukine-2 humaine comprenant le remplacement du reste cystéine en position 125, dans la numérotation correspondant à l'interleukine-2 native humaine, par un acide aminé neutre.

35. Sérine-$_{125}$ interleukine-2 mutéine recombinante humaine qui est incapable de former un pont disulfure intra-moléculaire non approprié mais qui est stable au stockage par rapport à l'interleukine-2 native réduite obtenue par voie bactérienne et qui a la séquence d'acides aminés déduite représentée sur la Figure 6b.

36. Formulation pharmaceutique ou vétérinaire comprenant une mutéine selon la revendication 35 formulée pour une utilisation pharmaceutique ou vétérinaire respectivement.

37. Utilisation d'une mutéine selon la revendication 35 pour fabriquer un médicament.

0 109 748

FIG. 1

0 109 748

FIG. 2

2

FIG. 3

FIG. 4

FIG. 5

```
          10         20         30         40         50         60
ATGCCTACTT CAAGTTCTAC AAAGAAAACA CAGCTACAAC TGGAGCATTT ACTGCTGGAT
          70         80         90        100        110        120
TTACAGATGA TTTTGAATGG AATTAATAAT TACAAGAATC CCAAACTCAC CAGGATGCTC
         130        140        150        160        170        180
ACATTTAAGT TTTACATGCC CAAGAAGGCC ACAGAACTGA AACATCTTCA GTGTCTAGAA
         190        200        210        220        230        240
GAAGAACTCA AACCTCTGGA GGAAGTGCTA AATTTAGCTC AAAGCAAAAA CTTTCACTTA
         250        260        270        280        290        300
AGACCCAGGG ACTTAATCAG CAATATCAAC GTAATAGTTC TGGAACTAAA GGGATCTGAA
         310        320        330        340        350        360
ACAACATTCA TGTGTGAATA TGCTGATGAG ACAGCAACCA TTGTAGAATT TCTGAACAGA
         370        380        390        400        410        420
TGGATTACCT TTTCTCAGAG CATCATCTCA ACACTGACTT GA
```

## FIG. 6 a

```
         5                  10                 15                 20
MetProThrSerSer SerThrLysLysThr GlnLeuGlnLeuGlu HisLeuLeuLeuAsp
         25                 30                 35                 40
LeuGlnMetIleLeu AsnGlyIleAsnAsn TyrLysAsnProLys LeuThrArgMetLeu
         45                 50                 55                 60
ThrPheLysPheTyr MetProLysLysAla ThrGluLeuLysHis LeuGlnCysLeuGlu
         65                 70                 75                 80
GluGluLeuLysPro LeuGluGluValLeu AsnLeuAlaGlnSer LysAsnPheHisLeu
         85                 90                 95                100
ArgProArgAspLeu IleSerAsnIleAsn ValIleValLeuGlu LeuLysGlySerGlu
        105                110                115                120
ThrThrPheMetCys GluTyrAlaAspGlu ThrAlaThrIleVal GluPheLeuAsnArg
        125                130                135                140
TrpIleThrPheSer GlnSerIleIleSer ThrLeuThr---
```

## FIG. 6 b